# EUROPÄISCHE PATENTSCHRIFT

(11) **EP 0 865 447 B1**
(45) Veröffentlichungstag und Bekanntmachung des Hinweises auf die Patenterteilung: **12.02.2003**
(21) Anmeldenummer: 96939113.5
(22) Anmeldetag: 25.11.1996
(51) Int. Cl.: C07K 7/64, C12P 21/04, A61K 38/12

(54) **CYCLISCHES DODECAPEPTID UND VERFAHREN ZU SEINER HERSTELLUNG**
CYCLIC DODECAPEPTIDE AND PROCESS FOR THE PREPARATION THEREOF
DODECAPEPTIDE CYCLIQUE ET SON PROCEDE DE PRODUCTION

(30) Priorität: 06.12.1995 DE 19545463
(43) Veröffentlichungstag der Anmeldung: 23.09.1998
(73) Patentinhaber: Bayer CropScience AG, 40789 Monheim (DE)
(72) Erfinder: ANKE, Heidrun, D-67663 Kaiserslautern (DE); ETZEL, Winfried, D-42799 Leichlingen (DE); GAU, Wolfgang, D-42113 Wuppertal (DE); HAIN, Rüdiger, D-40764 Langenfeld (DE); KILIAN, Michael, D-51379 Leverkusen (DE); MAYER, Anke, D-76829 Landau (DE); STERNER, Olov, S-212 42 Malmö (SE)
(86) Internationale Anmeldenummer: EP9605199
(87) Internationale Veröffentlichungsnummer: WO97020857

(56) Entgegenhaltungen:
- US-A- 4 117 118
- US-A- 4 252 795
- US-A- 4 764 503
- CHEMICAL ABSTRACTS, vol. 126, Columbus, Ohio, US; abstract no. 141834, MAYER, A. ET AL: "Screening of higher fungi for the production of nematicidal compounds using Meloidogyne incognita (Kofoid & White) chitwood as test organism" XP002027891 & MEDED. - FAC. LANDBOUWKD. TOEGEPASTE BIOL. WET. (UNIV. GENT) (1996), 61(3A), 839-847 CODEN: MFLBER, 1996,
- DATABASE WPI Section Ch, Week 8223 Derwent Publications Ltd., London, GB; Class B02, AN 82-46656E XP002027892 & JP 57 063 093 A (MITSUBISHI CHEM IND KK) , 16.April 1982

## Beschreibung

Die vorliegende Erfindung betrifft eine neue organisch-chemische Verbindung, welche im folgenden kurz Omphalotin genannt wird, ein Verfahren zu ihrer Herstellung auf im wesentlichen mikrobiologischem Wege und ihre Verwendung als Mikrobizid und Schädlingsbekämpfungsmittel, vorzugsweise zur Bekämpfung von tierischen Schädlingen, Schadpilzen und Bakterien.

Es wurde das neue Omphalotin gefunden, für das aufgrund der vorliegenden spektroskopischen und sonstigen analytischen Ergebnisse die folgende Formel (I) vorgeschlagen wird: 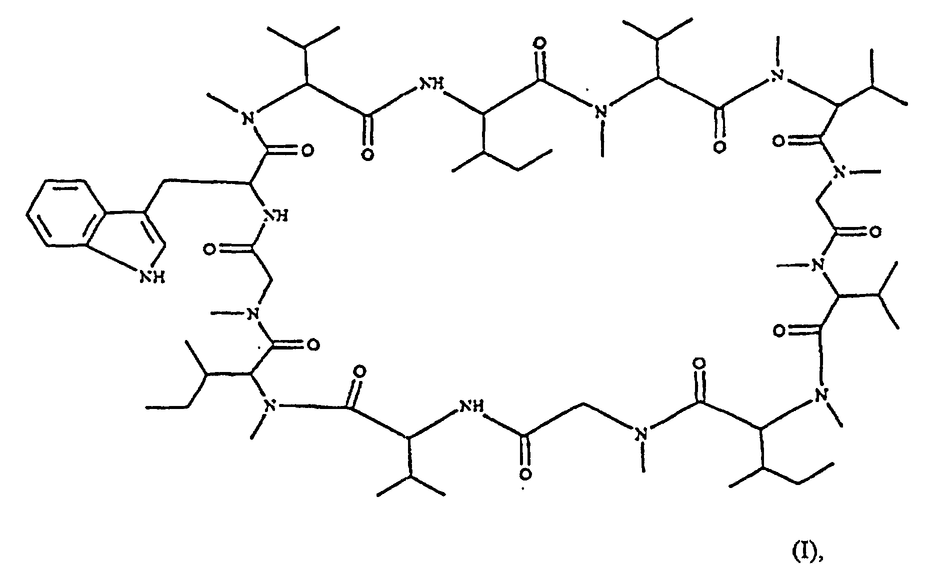 wobei ― (endständig) für -CH₃, 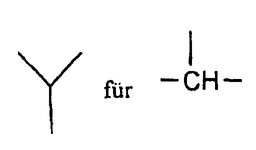 oder (endständig) für 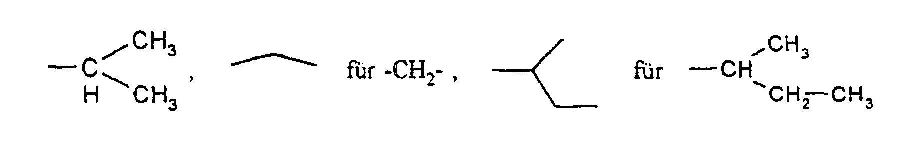 und 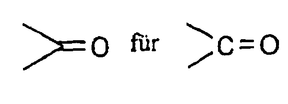 stehen.

Weiterhin wurde gefunden, daß das neue Omphalotin zur Bekämpfung von Schädlingen und Parasiten bei Pflanzen und Warmblütern verwendet werden kann. Es weist insbesondere eine hohe Wirksamkeit gegen Nematoden und Arthropoden (wie Insekten und Spinnentiere), sowie gegen mikrobielle Schädlinge, insbesondere gegen Pilze und Bakterien auf. Die neue Verbindung und diese Verbindung enthaltende Mittel können aufgrund dieser Eigenschaften besonders vorteilhaft im Pflanzenschutz, Vorratsschutz, im Hygienebereich sowie in der Tierzucht und Tierhaltung eingesetzt werden.

Die neue Verbindung der Formel (I) wird dadurch erhalten, daß man geeignete Mikroorganismen der Klasse Basidiomycetes, vorzugsweise aus den Gattungen Omphalotus und Lampteromyces, besonders bevorzugt Omphalotus, in üblicher Weise in einem Nährmedium, welches assimilierbare Kohlenstoff- und Stickstoffquellen sowie Mineralsalze enthält, unter aeroben Bedingungen kultiviert und die gewünschte Verbindung nach üblichen Methoden isoliert.

Bei Kenntnis der Eigenschaften der neuen erfindungsgemäßen Verbindung ist es mit Hilfe von üblichen chromatographischen, spektroskopischen, mikrobiologischen (z.B. Hemmhoftest) und/oder biologischen Methoden (z.B. durch Wirkungsbestimmungen gegen Nematoden oder Insekten) leicht und rasch möglich, in Routineverfahren solche geeigneten Mikroorganismenstämme auszusuchen, welche das erfindungsgemäße Omphalotin produzieren.

Für die mikrobiologische Herstellung der erfindungsgemäßen Verbindung werden bevorzugt Omphalotus-Stämme, insbesondere Omphalotus olearius-Stämme (synonym: Clitocybe illudens-Stämme), eingesetzt. Ganz besonders bevorzugt werden hierbei die Omphalotus olearius-Stämme Nr. 83 039, 90 173, 91 050, 92 095, 93 162 und 90 170 sowie die Varianten und Mutanten dieser Stämme, welche die für die Ausführung der vorliegenden Erfindung wesentlichen Merkmale aufweisen, bzw. die gleiche Funktion erfüllen, verwendet.

Die neuen Stämme können, wie folgt, beschrieben werden:
1. Vergleich der Morphologie des Myzels verschiedener Omphalotus olearius-Stämme auf Festmedium (M1)

| Pilzstamm | Myzelbeschaffenheit und -farbe |
|---|---|
| Omphalotus olearius 83 039 (DSM 9737) | Das zu Beginn des Wachstums weiße Myzel färbt sich nur schwach gelb, selten etwas bräunlich. Abscheidungen, wie bei den anderen Stämmen konnten nicht beobachtet werden. |
| | |
| Omphalotus olearius 90 170 (DSM 9742) | Das junge Myzel ist weiß-flauschig, das ältere Myzel färbt sich orange-schwarz. Vereinzelt kommt es zur Abscheidung von braungefärbten Tröpfchen. |
| | |
| Omphalotus olearius 90 173 (DSM 9738) | Das junge, weiße Myzel wächst weniger dicht und flauschig, als bei 90 170. Das ältere Myzel ist anfangs hellgelb und wird später orange-schwarz. Vereinzelt werden braune Abscheidungen beobachtet. |
| | |
| Omphalotus olearius 91 050 (DSM 9739) | Wachstum und Myzelfärbung sind ähnlich de von 90 170, die Abscheidungen von braunen Tropfen sind jedoch zahlreicher. |
| | |
| Omphalotus olearius 92 095 (DSM 9740) | Das junge, weiße Myzel wird intensiv orange, später leicht bräunlich. Die Abscheidung von braunen Tropfen ist sehr ausgeprägt. Das Myzel wird nicht schwarz. |
| | |
| Omphalotus olearius 93 162 (DSM 9741) | Das junge, gelbliche Myzel wird ähnlich 92 095 stark orange und scheidet ebenfalls viele braune Tropfen ab. |

2. Wachstum verschiedener Omphalotus olearius-Stamme, bei unterschiedlichen Temperaturen in 1 l Schüttelkultur Ml Medium in 2 l Erlenmeyerkolben

| Pilzstamm | Inkubationstemperatur [°C] | | |
|---|---|---|---|
| | 24 | 27 | 30 |
| Omphalotus olearius 83 039 (DSM 9737) | maximale Wachstumsrate | mittlere Wachstumsrate | stark reduziertes Wachstum |
| Omphalotus olearius 90 170 (DSM 9742) | Wachstum etwas geringer als bei 30°C | | maximale Wachstumsrate |
| Omphalotus olearius 90 173 (DSM 9738) | Bei allen Temperaturen gleiches Wachstum | | |
| Omphalotus olearius 91 050 (DSM 9739) | Bei allen Temperaturen gleiches Wachstum | | |
| Omphalotus olearius 92 095 (DSM 9740) | mittlere Wachstumsrate | maximale Wachstumsrate | niedrigste Wachstumsrate |
| Omphalotus olearius 93 162 (DSM 9741) | maximale Wachstumsrate | maximale Wachstumsrate | niedrigste Wachstumsrate |

3. Pelletform und -größe der verschiedenen Omphalotus olearius-Stämme bei Wachstum in Flüssigkultur (M1)

| Pilzstamm | Pelletform und -größe |
|---|---|
| Omphalotus olearius 83 039 (DSM 9737) | Der Pilz bildet feste Pellets von 0,5 bis 1,5 cm Durchmesser |
| | |
| Omphalotus olearius 90 170 (DSM 9742) | Der Pilz wächst in sehr kleinen, festen Pellets mit ca. 0,5 cm Durchmesser |
| | |
| Omphalotus olearius 90 173 (DSM 9738) | Der Pilz wächst in ähnlicher Pelletform und -größe wie 90 170 |
| | |
| Omphalotus olearius 91 050 (DSM 9739) | Die Pelletgröße variiert häufig, ist aber in der Regel etwas größer als bei 90 170 (0,5 bis 1,5 cm). Die Pellets sind "fransig". |
| | |
| Omphalotus olearius 92 095 (DSM 9740) | Der Pilz bildet die größten Pellets mit 1 bis 2 cm Durchmessern und "stachliger" Oberfläche. |
| | |
| Omphalotus olearius 93 162 (DSM 9741) | Der Pilz bildet Pellets mit bis zu 1,5 cm Durchmesser und weniger fester, eher "fransiger" Struktur. |

Die oben genannten Omphalotus olearius-Stamme sind neu. Sie wurden bei der Deutschen Sammlung von Mikroorganismen und Zellkulturen GmbH (DSM), Mascheroder Weg 1b, D 38124 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt und haben die folgenden Hinterlegungsnummern bzw. Eingangsnummern:

| Stamm | Hinterlegungs- bzw. Eingangsnummer | Hinterlegungsdatum |
|---|---|---|
| Omphalotus olearius Nr. 83 039 | DSM 9737 | 13.02.1995 |
| Omphalotus olearius Nr. 90 173 | DSM 9738 | 13.02.1995 |
| Omphalotus olearius Nr. 91 050 | DSM 9739 | 13.02.1995 |
| Omphalotus olearius Nr. 92 095 | DSM 9740 | 13.02.1995 |
| Omphalotus olearius Nr. 93 162 | DSM 9741 | 13.02.1995 |
| Omphalotus olearius Nr. 90 170 | DSM 9742 | 13.02.1995 |

Die Struktur des aus Omphalotus olearius isolierten Omphalotus wurde mit Hilfe von ¹H-, ¹³C-, COSY-, NOESY-, HMQC und HMBC-NMR-Spektren, sowie eines ESI-MS-Spektrums aufgeklärt. Es wurde eine Molmasses von 1318 Da bestimmt. Die NMR-Spektren wurden von einer Lösung von ca. 3 mg der Substanz in d₆-DMSO aufgenommen. Die ¹H- und ¹³C-NMR-Spektren wurden auf einem Bruker AMX-400-Gerät, die 2-D-Spektren auf einem Bruker DMX-600 aufgenommen. Die einzelnen Aminosäuren wurden mit Hilfe des COSY- und des HMQC-Spektrums zugeordnet. Mit Hilfe des COSY-Spektrums werden die Protonensignale der einzelnen Aminosäuren ausgehend von dem NH, bzw. dem α-Proton aufgrund der H-H-Kopplungen zugeordnet. Das HMQC-Spektrum ermöglicht dann die entsprechende Zuordnung der Kohlenstoffsignale. Die Sequenz der Aminosäuren wurde mit Hilfe des NOESY- und des HMBC-Spektrums zugeordnet. lm NOESY-Spektrum wird die räumliche Nähe des NH's einer Aminosäure zu dem (n) α-Proton(en) der nächsten Aminosäure detektiert. Im HMBC-Spektrum korrelieren die α-Protonen zweier verknüpfter Aminosäuren auf den gleichen Carbonylkohlenstoff. Die α-Protonen zeigen also zwei Korrelationsspeaks zu Carbonylkohlenstoffen: einen durch die Kopplung über zwei Bindungen zum Carbonylkohlenstoff der eigenen Aminosäure und einen über drei Bindungen zum Carbonylkohlenstoff der N-verknüpften Aminosäure.

Es wurde die folgende Struktur ermittelt:

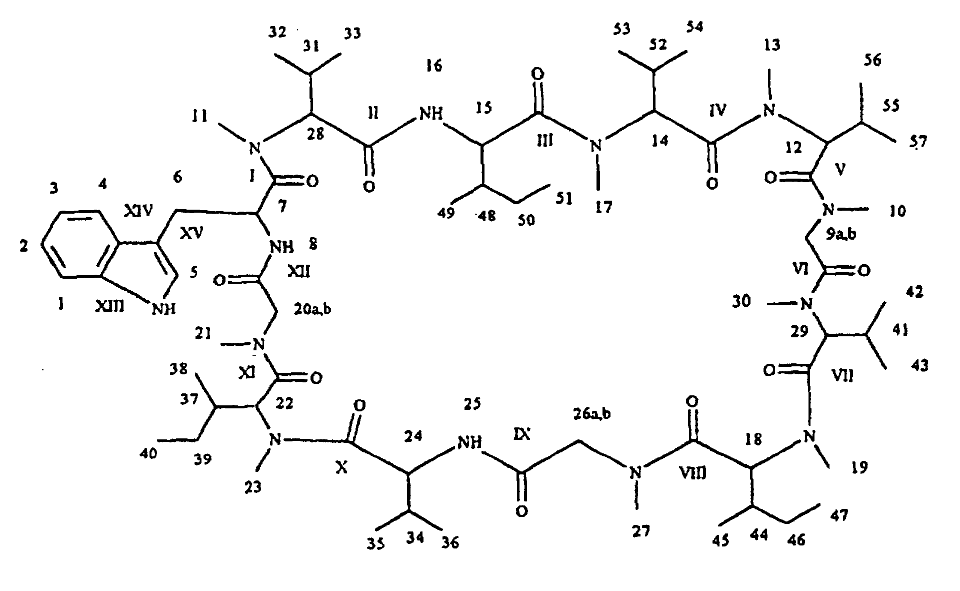

Die Zuordnung der ¹H- und ¹³C-Signale ist in den beiden folgenden Tabellen gegeben:

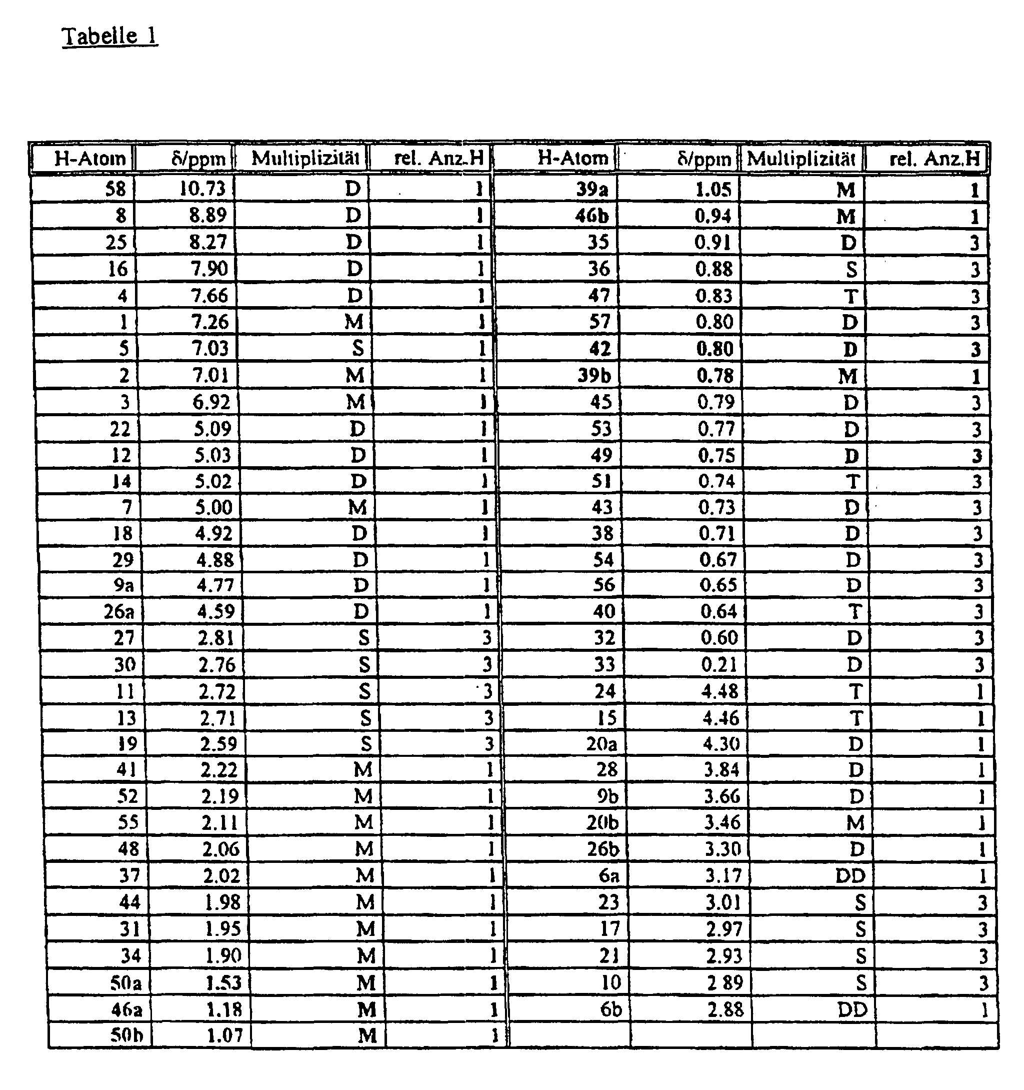

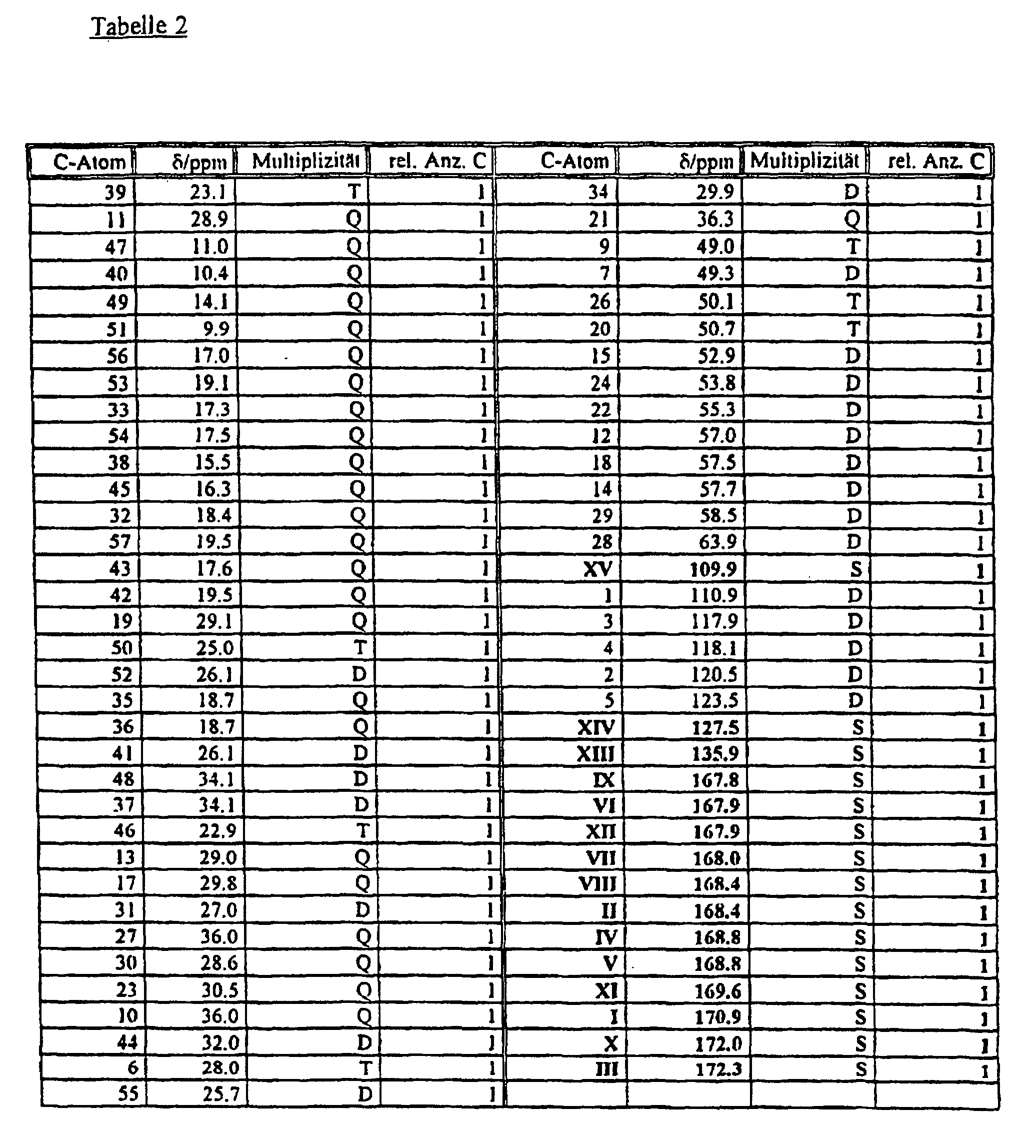

Die Struktur der neuen erfindungsgemäßen Verbindung wurde anhand von umfangreichen analytischen, insbesondere spektroskopischen Untersuchungen ermittelt. Da jedoch bei kompliziert gebauten Stoffen Irrtümer in der Interpretation der analytischen Befunde nicht immer völlig ausgeschlossen werden können, soll Omphalotin zusätzlich auch durch einige charakteristische physikalisch-chemische Daten sowie Spektren beschrieben werden:

| | | |
|---|---|---|
| a) | Aussehen | weißes Pulver |
| | | |
| b) | Löslichkeit | leicht löslich in Methanol, Aceton und 2-Propanol, weniger löslich in Essigsäureethylester und schwer löslich in Cyclohexan und t.-Butyl-methylether |
| | | |
| c) | Molgewicht | 1317 |
| | | |
| d) | Summenformel (Elementaranalyse) | C₆₉H₁₁₅N₁₃O₁₂ |
| | | |
| e) | Spektren | Die Daten der ¹H-NMR-Spektren sind aus den Tabellen 1 und 2 der Beschreibung ersichtlich |

Das neue Omphalotin wird erfindungsgemäß durch die Fermentation geeigneter Mikroorganismenstämme der Klasse Basidiomycetes, insbesondere der Ordnungen Omphalotus und Lampteromyces, vorzugsweise Omphalotus olearius (synonyme Bezeichnung: Clitocybe illudens) und ganz besonders bevorzugt der Omphalotus olearius Stämme 83 039 (DSM 9737), 90 173 (DSM 9738), 91 050 (DSM 9739), 92 095 (DSM 9740), 93 162 (DSM 9741) oder 90 170 (DSM 9742) oder deren Mutanten oder Varianten erzeugt

Das erfindungsgemäße Fermentationsverfahren wird in üblicher Weise durchgeführt. Es kann mit Hilfe fester, halbfester oder flüssiger Nährmedien durchgeführt werden. Bevorzugt werden wäßrig-flüssige Nährmedien verwendet.

Die Beimpfung der Nährmedien erfolgt nach allgemein üblichen Methoden, z.B. über Schrägrohrchen oder Kolbenkulturen.

Die Kultur erfolgt unter aeroben Bedingungen und kann gemäß den allgemein üblichen Methoden wie unter Verwendung von Schüttelkulturen z.B. in Schüttelkolben, von luftbewegten Kulturen oder von Submerskulturen durchgeführt werden. Bevorzugt erfolgt die Kultivierung im aeroben Submersverfahren in belüfteten Fermentern, z.B. in üblichen Submersfermentationstanks. Es ist möglich, die Kulturen kontinuierlich oder diskontinuierlich durchzuführen. Vorzugsweise wird diskontinuierlich gearbeitet.

Die Kultur kann in allen Nährmedien durchgeführt werden, welche bekannterweise zur Kultivierung von Mikroorganismen der Klasse Basidiomycetes verwendet werden. Das Nährmedium muß eine oder mehrere assimilierbare Kohlenstoffquellen und Stickstoffquellen sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können. Als Kohlenstoffquellen kommen alle üblichen Kohlenstoffquellen in Frage. Beispielsweise seien Kohlenhydrate, insbesondere Polysaccharide, wie Stärke oder Dextrine, Disaccharide, wie Maltose oder Rohrzucker, Monosaccharide wie Glukose oder Xylose, Zuckeralkohole wie Mannit oder Glycerin sowie natürlich vorkommende Gemische wie Malzextrakt, Melasse oder Molkepulver genannt. Als Stickstoffquellen kommen alle üblichen organischen und anorganischen Stickstoffquellen in Frage. Beispielsweise seien Eiweißstoffe, Eiweißhydrolysate, Aminosäuren wie Glutaminsäure, Asparaginsäure, Arginin, Lysin, Ornithin oder Serin, Nucleosidbasen wie Cytosin oder Uracil sowie Sojabohnenmehl, Baumwollsamenmehl, Linsenmehl, Erbsenmehl, löslich und unlösliche, pflanzliche Proteine, Maisquellenwasser, Hefeextrakt, Peptone und Fleischextrakt sowie Ammoniumsalze und Nitrate, z.B. NH₄Cl, (NH₄)₂SO₄, Na NO₃ und KNO₃ aufgeführt. Die Mineralsalze, welche im Nährmedium enthalten sein sollten, liefern z.B. folgende Ionen:
Mg⁺⁺, Na⁺, K⁺, Ca⁺⁺, NH₄⁺, Cl⁻, SO₄⁻⁻, PO₄⁻⁻⁻ und NO₃⁻
sowie Ionen der üblichen Spurenelemente, wie Cu, Fe, Mn, Mo, Zn, Co, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen. Im allgemeinen enthalten die Nährlösungen vorzugsweise etwa 0,5 bis 8 %, insbesondere 0,6 bis 6 % Kohlenstoffquellen, vorzugsweise etwa 0,5 bis 4 %, insbesondere 0,5 bis 2 % Stickstoffquellen und vorzugsweise etwa 0,001 bis 0,5 %, insbesondere 0,003 bis 0,3 % Mineralsalze.

Bei der Durchführung des Verfahrens kann es günstig sein, zu Beginn der Kultivierung nur relativ geringe Konzentrationen der löslichen Nährlösungsbestandteile zu verwenden und dann im Laufe der ersten 3 Kultivierungstage durch häufigere Zusätze diese Bestandteile in Form steriler, relativ konzentrierter Lösung dem Kulturansatz fraktioniert zuzufüttern.

Der pH-Wert der wachsenden Kulturen sollte vorzugsweise zwischen etwa 5 und etwa 10, insbesondere zwischen 3,0 und 8,0 gehalten werden. Ein zu starker pH-Abfall in den sauren Bereichen kann durch Zusätze einer organischen oder anorganischen Base, vorzugsweise von CaCO₃ vermieden werden. Wie in der Fermentationstechnologie üblich, kann auch eine automatische pH-Regulierung durchgeführt werden, bei der sterile organische oder anorganische Säure, z.B. H₂SO₄, oder sterile Lauge, z.B. NaOH in Abständen in die Kulturlösung eingespritzt wird.

Es ist zweckmäßig sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 15 und etwa 40°C, bevorzugt zwischen 20 und 35°C liegen, besonders bevorzugt liegt sie zwischen etwa 22 und 27°C. Die Dauer der Züchtung kann stark variiert werden, wobei z.B. die Zusammensetzung des Nährmediums und die Züchtigungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden erfindungsgemäßen Verbindung im allgemeinen ihr Maximum etwa 1 bis 10, vorzugsweise etwa 4 bis 7 Tage nach Züchtungsbeginn erreicht. Das gewünschte Endprodukt der Fermentation kann mit Hilfe von dünnschichtchromatographischen Untersuchungen, mit Hilfe von HPLC und UV-Absorptionsspektren im Plattendiffusionstest mit einem geeigneten Pilz als Teststamm oder anhand der nematiziden oder insektiziden Wirksamkeit bestimmt werden.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Vorrichtungen können z.B. die Dampf- als auch die Trockensterilisation verwendet werden, wobei die Temperaturen vorzugsweise bei 100 bis 140°C, insbesondere bei 120 bis 130°C liegen können.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöle, Polyoxyethylen- bzw. Polyoxypropylenverbindungen (z.B. in Mengen bis etwa 1 %) zugesetzt werden. Schaum kann auch mit Hilfe der üblichen mechanischen Vorrichtungen (welche z.B. Zentrifugalkräfte benutzen) gedämpft oder beseitigt werden.

Die erfindungsgemäße Verbindung kann aus dem Kulturmedium und aus der Biomasse nach allgemein üblichen physikalisch-chemischen Methoden isoliert werden. Die Isolierung kann z.B. gemäß den üblichen Extraktionsverfahren, Fällungsverfahren und/oder Chromatographieverfahren erfolgen. Der isolierte Stoff kann mit Hilfe der genannten Methoden auch feingereinigt werden. Für viele Fälle ist jedoch eine Feinreinigung nicht erforderlich, da gegebenenfalls vorhandene geringfügige Verunreinigungen die Wirksamkeit der Verbindung nicht nachteilig beeinflußt.

Um bei den oben angegebenen Isolierungs- und Reinigungsmethoden die Fraktionen herauszufinden, in welchen die erfindungsgemäße Verbindung in höchster Konzentration bzw. Reinheit vorliegt, können die üblichen physikalisch-chemischen Methoden, z.B. Messen einer charakteristischen Bande im Spektrum oder der R_{f}-Werte, Bestimmung der antimikrobiellen oder der nematiziden und insektiziden Aktivität usw. herangezogen werden. Diese Methoden können auch verwendet werden, um in Routineverfahren geeignete Mikroorganismen aufzufinden.

Die Isolierung und Reinigung der erfindungsgemäßen Verbindung kann z.B. im Falle, daß ein flüssiges wäßriges Nährmedium verwendet wird, wie folgt vorgenommen werden:

Nach seiner Anreicherung im Kulturüberstand werden Kulturfiltrat und Mycel mit üblichen Methoden separiert (z.B. Zentrifugation).

Aus dem Kulturfiltrat, vorzugsweise aus der Biomasse, kann die erfindungsgemäße Verbindung mit Hilfe von üblichen Extraktionsverfahren, Fällungsverfahren und/oder Chromatographieverfahren isoliert und gegebenenfalls gereinigt werden. Die Chromatographie kann in Form der Säulenchromatographie durchgeführt werden.

Als Adsorptionsmittel können die üblichen anorganischen oder organischen Adsorptionsmittel eingesetzt werden, wie z.B. Aluminiumoxid, Kieselgel, Magnesiumsilikat, Aktivkohle, Cellulose, Cellulosederivate, Kunstharze wie Polyamide, z.B. acetyliertes Polyamid, Dextrangele oder modifizierte Dextrangele. Als Laufmittel können die verschiedensten Lösungsmittel oder Lösungsmittelgemische verwendet werden, in welchen die erfindungsgemäße Verbindung löslich ist. Bevorzugt werden Wasser, Ammoniaklösung, Chloroform und Methanol oder ihre Gemische (beispielsweise Gemische aus Chloroform, Methanol und wäßrigem NH₃ oder Methanol und Wasser) eingesetzt.

Bevorzugt werden zur Isolierung der erfindungsgemäßen Verbindung Chromatographie-Verfahren verwendet, z.B. unspezifische Adsorption an Sorbentien wie Kieselgel, Ionenaustauschchromatographie oder Geldiffusionschromatographie. Dies sind die von der Reinigung wasserlöslicher, geladener Naturstoffe her bekannten Methoden.

Weiterhin kann vorteilhaft auch die Methode der Craig-Verteilung (flüssig-flüssig-Verteilung) eingesetzt werden.

Aus ihren Lösungen kann die erfindungsgemäße Verbindung nach den üblichen Methoden, z.B. Verdampfen des Lösungsmittels, Gefriertrocknung usw. erhalten werden.

In einer bevorzugten Ausführungsform der Erfindung wird aus dem bei der aeroben Kultur der Stämme bei etwa 27°C erhaltenen Fermentationsgut (Kulturbrühe und Mycel) durch Zentrifugation die Biomasse (das Mycel) gewonnen.

Der neue Stoff wird vorzugsweise durch Extraktion der Biomasse gewonnen. Er kann auch aus dem Kulturfiltrat durch Adsorption an Aktivkohle bzw. an geeignete Harze isoliert werden. Als ökonomischste Methode erwies sich die Bindung des erfindungsgemäßen Stoffes an unspezifische Adsorberharze auf Polystyrolbasis (z.B. Amberlite XAD oder Lewatit OC 1031). Die Desorption des erfindungsgemäßen Stoffes wird fraktioniert, durch Mischungen aus Wasser und organischen Lösungsmitteln, insbesondere Wasser/Methanol vorgenommen. Die durch den Test gegen Meloidogyne incognita ermittelten aktiven Fraktionen werden unter reduziertem Druck bis zur vollständigen Entfernung des organischen Lösungsmittels konzentriert und gegebenenfalls gefriergetrocknet.

Das gefriergetrocknete Rohprodukt wird in Wasser aufgenommen und nach dem Abtrennen der unlöslichen Bestandteile durch übliche chromatographische Verfahren weiter gereinigt. Bevorzugt werden dabei eine erneute Bindung an Adsorberharze (z.B. Lewatit OC 1031), eine weitere Reinigung der aktiven Fraktionen durch Chromatographie (z.B. Sephadex LH 20). Der neue Stoff wird schließlich durch übliche chromatographische Methoden, vorzugsweise durch Chromatographie an Kieselgel oder präparative HPLC, rein dargestellt.

Die erfindungsgemäße Verbindung eignet sich zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitaren Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Zu den mikrobiellen Schädlingen, die mit Hilfe der neuen Verbindung bekämpft werden können, gehören insbesondere die phytopathogenen Pilze:

Plasmodiophoromycetes, Oomycetes, Chytridiomycetes, Zygomycetes, Ascomycetes, Basidiomycetes, Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
   Pseudomonas-Arten, wie beispielsweise Pseudomonas syringae pv. lachrymans;
   Erwinia-Arten, wie beispielsweise Erwinia amylovora;
   Pythium-Arten, wie beispielsweise Pythium ultimum;
   Phytophthora-Arten, wie beispielsweise Phytophthora infestans;
Pseudoperonospora-Arten, wie beispielsweise Pseudoperonospora humuli oder Pseudoperonospora cubense;
   Plasmopara-Arten, wie beispielsweise Plasmopara viticola;
   Peronospora-Arten, wie beispielsweise Peronospora pisi oder P. brassicae;
Erysiphe-Arten, wie beispielsweise Erysiphe graminis;
   Sphaerotheca-Arten, wie beispielsweise Sphaerotheca fuliginea;
   Podosphaera-Arten, wie beispielsweise Podosphaera leucotricha;
   Venturia-Arten, wie beispielsweise Venturia inaequalis;
   Pyrenophora-Arten, wie beispielsweise Pyrenophora teres oder P. graminea
   (Konidienform: Drechslera, Syn: Helminthosporium);
Cochliobolus-Arten, wie beispielsweise Cochliobolus sativus
   (Konidienform: Drechslera, Syn: Helminthosporium);
   Uromyces-Arten, wie beispielsweise Uromyces appendiculatus;
Puccinia-Arten, wie beispielsweise Puccinia recondita;
   Tilletia-Arten, wie beispielsweise Tilletia caries;
   Ustilago-Arten, wie beispielsweise Ustilago nuda oder Ustilago avenae;
   Pellicularia-Arten, wie beispielsweise Pellicularia sasakii;
   Pyricularia-Arten, wie beispielsweise Pyricularia oryzae;
   Fusarium-Arten, wie beispielsweise Fusarium culmorum;
Botrytis-Arten, wie beispielsweise Botrytis cinerea;
   Septoria-Arten, wie beispielsweise Septoria nodorum;
   Leptosphaeria-Arten, wie beispielsweise Leptosphaeria nodorum;
   Cercospora-Arten, wie beispielsweise Cercospora canescens;
   Alternaria-Arten, wie beispielsweise Alternaria brassicae;
   Pseudocercosporella-Arten, wie beispielwseise Pseudocercosporella herpotrichoides. Weiterhin sei Helminthosporium carbonum aufgeführt.

Die erfindungsgemäße Verbindung der Formel (I) zeichnet sich insbesondere durch eine hervorragende nematizide Wirkung aus, beispielsweise gegen Meloidogyne incognita.

Sie zeigt systemische Wirkung und kann auch über das Blatt angewendet werden.

Der Wirkstoff kann in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen des Wirkstoffes mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsaureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl)-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Der erfindungsgemäße Wirkstoff kann ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew -% liegen.

Die Anwendung geschieht in einer den Anwendungsformen angepaßten üblichen Weise. Der Wirkstoff kann an oberirdische Pflanzenteile oder über den Boden appliziert werden. Auch eine Behandlung von Saatgut ist möglich.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Der Wirkstoff eignet sich bei günstiger Warmblütertoxizitat auch zur Bekämpfung von pathogenen Endoparasiten, die bei Menschen und insbesondere in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:
Aus der Ordnung der Pseudophyllidea z.B.: Diphyllobothrium spp., Spirometra spp., Schistocephalus spp., Ligula spp., Bothridium spp., Diphlogonoporus spp..
Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..
Aus der Unterklasse der Monogenea z.B.: Gyrodactylus spp., Dactylogyrus spp., Polystoma spp..
Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..
Aus der Ordnung der Enoplida z.B.: Trichuris spp., Capillaria spp., Trichomosoides spp., Trichinella spp..
Aus der Ordnung der Rhabditia z.B.: Micronema spp., Strongyloides spp..
Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..
Aus der Ordnung der Oxyurida z.B.: Oxyuris spp., Enterobius spp., Passalurus spp., Syphacia spp., Aspiculuris spp., Heterakis spp..
Aus der Ordnung der Ascaridia z.B.: Ascaris spp., Toxascaris spp., Toxocara spp., Parascaris spp., Anisakis spp., Ascaridia spp..
Aus der Ordnung der Spirurida z.B.: Gnathostoma spp., Physaloptera spp., Thelazia spp., Gongylonema spp., Habronema spp., Parabronema spp., Draschia spp., Dracunculus spp..
Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..
Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung des Wirkstoffes erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung des Wirkstoffes geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: PHysiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Der Wirkstoff läßt sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfordernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Ole, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl /Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Die Herstellung des neuen Omphalotin sowie sein biologischen Eigenschaften sollten anhand der folgenden Beispiele erläutert werden.

Zu den Beispielen (wie auch in der übrigen Beschreibung) beziehen sich, wo nichts anderes angegeben wird, %-Angaben auf Gewichtsprozente und die Angaben zu Lösungsmittelgemischen auf Volumenteile.

Die in den Beispielen aufgeführten Testmethoden können auch besonders günstig dazu eingesetzt werden, bei der Produktion von Omphalotin die Fraktionen zu bestimmen, welche das Omphalotin enthalten. Sie können dabei auch zur Abschätzung des jeweiligen Omphalotin-Gehaltes herangezogen werden.

### Beispiel 1 (Herstellung von Omphalotin durch Fermentations von Omphalotus olearius Nr. 90 170 = DSM 9742)

### 1.1 Herstellung Festmedienagarplatten zur Kultur von Omphalotus olearius

### 1.2 Kultivierung von Omphalotus olearius auf Festmedienagarplatten

### 1.3 Herstellung des Nährmediums für die Vorkultur von Omphalotus olearius

### 1.4 Beimpfen und Kultur von Omphalotus olearius im Vorkulturmedium

### 1.5 Herstellung des Nährmediums zur Fermentation von Omphalotus olearius im Vorfermenter

### 1.6 Beimpfen und Kultur von Omphalotus olearius im Vorfermenter

### 1.7 Herstellung des Nährmediums für die Fermentation von Omphalotus olearius im Hauptfermenter

### 1.8 Beimpfen und Kultur von Omphalotus olearius im Hauptfermenter

### 1.8 Ernte der Biomasse nach der Fermentation

### 1.1 Herstellung der Festmedienagarplatten zur Kultur von Omphalotus olearius

Zur Herstellung von Festmedienagarplatten werden

| | |
|---|---|
| 4 g/L | Glucose |
| 4 g/L | Hefeextrakt (Fa. Merck Nr. 3753, 64271 Darmstadt, Germany) |
| 10 g/L | Löflund's Gerstenmalzextrakt (Fa. Dr. Fränkle & Max Eck, 70736 Fellbach, Germany) |
| 20 g/L | Agar Agar (Fa. Merck Nr. 1615, 64271 Darmstadt, Germany) |

eingewogen, in entmineralisiertem Wasser gelöst und unter Zugabe von Säure (c(HCl)=2 mol/L) auf einen pH-Wert von 5,5 eingestellt. Ein 2 1 Erlenmeyerkolben wird mit 1 I der oben aufgeführten Lösung befüllt, mit einem Wattestopfen verschloßen und in einem Autoklav (Fa. MMM, Typ LSS 666-1) 30 Minuten bei einer Temperatur von 121°C sterilisiert.

Unter ständigem Rühren wird die Lösung auf eine Temperatur von ca. 50 bis 60°C abgekühlt. 20 ml der Lösung werden unter sterilen Bedingungen in 90 mm Petrischalen überführt. Nach Erkalten der Lösung können die befüllten Petrischalen bei Raumtemperatur für maximal 8 Wochen vor einer Beimpfung gelagert werden.

### 1.2 Kultivierung von Omphalotus olearius auf Festmedienagarplatten

Der Agar einer, mit Omphalotus olearius (im vorliegenden Beispiel Nr. 90 170) gut bewachsenen Festmedienagarplatte, wird unter sterilen Bedingungen mit Hilfe eines Skalpells in 1 x 1 cm große Stücke unterteilt. Je ein Agar-Myzelstück wird mit einer Pinzette auf eine wie unter 1.1 beschriebene, Festmedienagarplatte überführt (Myzelseite nach oben) und in einem Inkubationsschrank (Fa. Heraeus, Typ BK 5060 E) bei 27°C für maximal 3 Wochen im Dunkeln inkubiert. Nach Ablauf von 3 Wochen können die bewachsenen Platten als Animpfplatten für die Fermentation oder zum erneuten Beimpfen von Festmedienagarplatten genutzt werden.

### 1.3 Herstellung Nährmedium für die Vorkultur von Omphalotus olearius

Es werden

| | |
|---|---|
| 4 g/L | Glucose |
| 4 g/L | Hefeextrakt (Fa. Merck Nr. 3753, 64271 Darmstadt, Germany) |
| 10 g/L | Löflund's Gerstenmalzextrakt (Fa. Dr. Fränkle & Max Eck, 70736 Fellbach, Germany) |

eingewogen, in entmineralisiertem Wasser gelöst und unter Zugabe von Säure (c(HCl)=2 mol/L) auf einen pH-Wert von 5,5 eingestellt. Ein 1 I Erlenmeyerkolben wird mit 300 ml der obengenannten Lösung befüllt, mit einem Wattestopfen verschloßen und 30 Minuten bei 121°C autoklaviert. Nach Abkühlung werden die befüllten Erlenmeyerkolben bei Raumtemperatur maximal für 8 Wochen vor einer Beimpfung gelagert.

### 1.4 Beimpfen und Kultur von Omphalotus olearius im Vorkulturmedium

Je 300 ml der unter 1.3 beschriebenen Nährlösung werden mit einer halben, gut bewachsenen, 2 bis 3 Wochen alten Festmedienplatte beimpft. Dazu werden 2 bis 3 Wochen alte Festmedienplatten unter sterilen Bedingungen mit einem Skalpell geteilt und mit einer Pinzette in einem Waring Mixer Aufsatz (Fa. Waring Blender, Typ 32BL79) überführt. Diese werden mit 50 ml sterilen destilliertem Wasser pro Festmedienplatte versetzt und 30 Sekunden lang homogenisiert (15 Sekunden Einstellung low, 15 Sekunden Einstellung high). Das Gesamtvolumen der homogenisierten Flüssigkeit wird mit einer 10 ml Pipette (Fa. Becton Dickinson and Company, Typ Falcon) gleichmäßig auf die zum Animpfen vorbereiteten Erlenmeyerkolben verteilt. Die Inkubation der Vorkulturkolben erfolgt im Schüttelschrank (Fa. Braun Melsungen Typ BS 4) bei 27°C Temperatur und einer Schüttelgeschwindigkeit von 120 UPM für 5 Tage.

### 1.5 Herstellung des Nährmediums zur Fermentation von Omphalotus olearius im Vorfermenter

Ein 42 L Vorfermenter (Fa. Braun Melsungen Typ Biostat P) der mit 30 L entmineralisiertem Wasser gefüllt ist, wird mit

| | |
|---|---|
| 4 g/L | Glucose |
| 4 g/L | Hefeextrakt (Fa. Merck Nr. 3753, 64271 Darmstadt, Germany) |
| 10 g/L | Löflund's Gerstenmalzextrakt (Fa. Dr. Fränkle & Max Eck, 70736 Fellbach, Germany) |
| 0,25 ml/L | Antischaummittel (Fa. Bayer, Baysilone E, 51371 Leverkusen, Germany) |

versetzt. Nach dem Befüllen wird der Fermenter verschlossen und der Inhalt 30 Minuten lang bei einer Temperatur von 121°C sterilisiert. Nach Abkühlen auf eine Betriebstemperatur von 27°C, wird ein Überdruck von 0,3 bar und eine Belüftungsrate von 0,25 vvm eingestellt. Nach Erreichen der max. Sauerstoffsättigung, wird das pO₂-Meter auf einen Ausgangswert von 99 % kalibriert.

Der pH-Wert des Mediums wird mit Hilfe von Säure (c(HCl)=2 mol/L) auf pH 5,5 eingestellt.

### 1.6 Beimpfen und Kultur von Omphalotus olearius im Vorfermenter

Der Vorfermenter wird mit 600 ml der unter 1.4 erstellten Vorkultur angeimpft. Dazu werden zwei 1 l Erlenmeyerkolben, die je 300 ml Vorkultur enthalten, unter sterilen Bedingungen in eine 2 l Glasflasche (Fa. Schott) überführt (die Flasche ist über einen Siliconschlauch mit einer Anstechgarnitur der Fa. Braun Melsungen verbunden).

Der Vorfermenter wird mittels dieser Anstechgarnitur über eine Membran im Deckel mit der Vorkultur steril beimpft.

| Fermentationsbedingungen: | |
|---|---|
| Temperatur | 27°C |
| Ruhrgeschwindigkeit | 200 UPM |
| Belüftungsrate | 0,25 vvm |
| Druck | 0,3 bar |
| pH-Wert | freilaufend |
| Antischaum | nach Bedarf |
| Inkubationszeit | ca. 120 h bzw. maximal bis zu dem Zeitpunkt, wo die Glucosekonzentration auf c(Glucose) ≤ 1 g/L gesunken ist. |

### 1.7 Herstellung des Nährmediums für die Fermentation von Omphalotus olearius im Hauptfermenter

Ein 450 L Fermenter (Fa. Braun Melsungen Typ Biostat 450 D), der mit 320 L entmineralisiertem Wasser gefüllt ist, wird auf das Arbeitsvolumen von 350 L bezogen mit

| | |
|---|---|
| 4 g/L | Glucose |
| 4 g/L | Fermtech Hexeextrakt (Fa. Merck Nr. 11926, 64271 Darmstadt, Germany) |
| 10 g/L | Löflund's Gerstenmalzextrakt (Fa. Dr. Fränkle & Max Eck, 70736 Fellbach, Germany) |
| 0,25 ml/L | Antischaummittel (Fa. Bayer, Baysilone E, 51371 Leverkusen, Germany) |

versetzt. Nach dem Befüllen wird der Fermenter verschlossen und der Inhalt 30 Minuten lang bei einer Temperatur von 121°C sterilisiert. Nach Abkühlen auf eine Betriebstemperatur von 27°C wird ein Überdruck von 0,3 bar und eine Belüftungsrate von 0,25 vvm eingestellt. Nach Erreichen der maximalen Sauerstoffsättigung wird das pO₂-Meter auf einen Ausgangswert von 99 % kalibriert.

Der pH-Wert des Mediums wird mit Hilfe von Säure (c(HCl)= 2 mol/L) auf pH 5,5 eingestellt.

### 1.8 Beimpfen und Kultur von Omphalotus olearius im Hauptfermenter

Nach Absinken der Glucosekonzentration c(Glucose) ≤ 1 g/L im Kulturfiltrat des Vorfermenters (siehe 1.6) wird der Hauptfermenter mit dem Inhalt des Vorfermenters über eine sterile Transferleitung beimpft. Die Glucosebestimmung erfolgt mit einem Beckmann Glucose Analyser.

| Fermentationsbedingungen: | |
|---|---|
| Temperatur | 27°C |
| Rührgeschwindigkeit | 150 UPM |
| Belüftungsrate | 0,25 vvm |
| Druck | 0,3 bar |
| pH-Wert | freilaufend |
| Antischaum | nach Bedarf |
| Inkubationszeit | 72 h bis 96 h |

### 1.9 Ernte der Biomasse nach der Fermentation

Bei einer Glucosekonzentration von c(Glucose) ≤ 1 g/L wird die Fermentation im 450 L Fermenter beendet und die Biomasse geerntet. Der Inhalt des Hauptfermenters wird über Gaze (Fa. Holthaus Medical, Verbandmull, Remscheid 11, Germany) von dem Nährmedium separiert. Die abfiltrierte Biomasse wird mehrmals mit entmineralisiertem Wasser gewaschen und anschließend in einer Trockenschleuder (Fa. Miele, Typ MZ 5942, Germany) bei einer Umdrehung von 4000 UPM 3 Minuten lang geschleudert. Es werden 3 bis 6 kg feuchte Biomasse geerntet. Die gewonnene Biomasse von Omphalotus olearius wird bei einer Temperatur von -34°C eingefroren und dort bis zur weiteren Aufarbeitung gelagert.

Die Fermentation der übrigen Omphalotus Stämme kann entsprechend dem vorstehenden Beispiel durchgeführt werden.

### Beispiel 2 (Isolierung und Reinigung von Omphalotin)

### 2.1 Herstellung des Rohextraktes des Wirkstoffes aus Omphalotus olearius und Anreicherung mittels Flüssig-Flüssig-Verteilung (Craig-Verteilung)

Ca. 7 kg Myzel-Pellets (Biomasse) aus dem nach Beispiel 1 gewonnenen Myzel wurden portionsweise homogenisiert und zweimal mit je 20 l Aceton digeriert. Anschließend wurde am Rotationsverdampfer unter Vakuum eingeengt. Zum Rückstand wurden 3 l Wasser und 3 l Essigsäureethylester zugegeben und gerührt. Die Oberphase wurde abgetrennt und das Lösungsmittel im Rotationsverdampfer unter Vakuum abdestilliert, die Unterphase wurde verworfen. Der Rückstand der Destillation wurde zweimal mit je 2 l Acetonitril und 0,5 I n-Heptan aufgenommen. Die nach Schütteln der Mischungen und Stehenlassen gebildeten Unterphasen wurden bei ca. 40°C eingedampft. Der verbliebene Rückstand wurde in die Craig-Verteilung eingesetzt, die wie folgt, durchgeführt wurde.

| Parameter für die Craig-Verteilung: | |
|---|---|
| Apparatur | 25 ml Apparatur (Fa. Labortec, Bubendorf, Schweiz) |
| Anzahl Verteilungselemente | 200 |
| Verteilungssystem | Essigester/n-Heptan/DMF (Dimethylformamid)/Wasser (2,5 / 7,5 / 5 / 5 v/v) |
| Phasenverhältnis | 1:1 |
| Trennstufen | n = 198 |
| Einsatz | 945 mg in zwei Elemente |
| Aufarbeitung | Nach Beendigung des Verteilungszyklus (n = 198) wurde der Inhalt jedes 10. Elementes entnommen, die Lösungsmittel am Rotationsverdampfer unter Vakuum abdestilliert, der Rückstand nach Wägung in 10 ml Methanol aufgenommen und mittels analytischer HPLC untersucht (siehe Beispiel 2.4). Das gesuchte Omphalotin befand sich in den Elementen E 48-87. Der Inhalt der Elemente wurde entnommen und vereinigt. Nach Zugabe von 2 l Wasser wurde geschüttelt und die Oberphase abgetrennt. Die Unterphase wurde jeweils 3 mal mit je 1 l Essigester p.a. extrahiert. Die vereinigten Oberphasen wurden anschließend 4 mal mit je 0,5 l Wasser extrahiert, und das Lösungsmittel am Rotationsverdampfer unter Vakuum abdestilliert. Die Ausbeute des zurückbleibenden aufkonzentrierten Rohextraktes betrug 135 mg. Die weitere Aufreinigung erfolgt nach Beispiel 2.3. |

### 2.2 Präparation des Rohextraktes des Wirkstoffes aus Omphalotus Olearius und Anreicherung des Wirkstoffes nach dem Prinzip der Gelfiltration

400 g lyophilisiertes Mycel wurden 2 Stunden unter Rühren mit 10 l Methanol extrahiert. Nach dem Entfernen des Lösungsmittels am Rotationsverdampfer erhielt man 35 g Rohextrakt. Dieser Rohextrakt wird in Portionen a 10 g gesplittet.

10 g Rohextrakt werden mit 5 ml Methanol gelöst und über eine Trennsäule (Höhe: 62 cm, Durchmesser: 5 cm), die mit Sephadex LH-20 (Pharmacia, Upsalla, Schweden)) gefüllt ist, mit Methanol als mobiler Phase chromatographiert. Die Flußrate wird dabei so eingestellt, daß 5,5 ml/min an Eluat gewonnen wird. Das Eluat wird in 100 ml Portionen fraktioniert aufgesammelt. Die Fraktionen, die Omphalotin enthalten, werden vereinigt und am Rotationsverdampfer eingedampft. Man erhält 2 g angereicherten Extrakt aus den oben eingesetzten 10 g. Die 2 g angereicherter Extrakt werden in Portionen zu 200 mg aufgeteilt.

Von dem oben angereicherten Extrakt werden 200 mg in 1 ml Methanol gelöst und auf eine zweite Trennsäule aufgegeben (Höhe 71,5 cm, Durchmesser 2,5 cm, Sephadex LH-20). Es wird jetzt mit einer Flußrate von 3 ml/min chromatographiert. Das Eluat wird in 10 ml Fraktionen aufgefangen. Diese Fraktionen werden ebenfalls mittels analytischer HPLC (siehe 2.4) auf Omphalotin hin untersucht. Das Omphalotin kann alternativ aber auch mittels eines Biotests z.B. nach Beispiel B detektiert werden. Die Fraktionen die Omphalotin enthalten werden vereinigt und am Rotationsverdampfer eingedampft. Man erhält 60 mg weiter angereicherten Extrakt aus den oben eingesetzten 200 mg. Die weitere Aufreinigung erfolgt nach 2.3.

### 2.3 Feintrennung der aus der Flüssig-Flüssig-Verteilung und der chromatographisch Trennungen erhaltenen angereicherten Wirkstoffraktionen mittels präparativer HPLC

Gerät: Jasco PU-980 mit Hochdruckpumpe LG-980-02-Gradientenmischer mit Multiwavelengthdetektor MD-910 (Gross-Umstadt, Deutschland)
Säule: Hibar RT Lichrospher RP 18 WP 300® (12 µm Partikeldurchmesser),
Dimension: 250 mm Säulenlänge * 25 mm Innendurchmesser
Eluent: A = Wasser, B = Acetonitril
Fluß: 5 ml/min
Detektion: UV, λ = 210 nm
Injektionsmenge: 100 mg gelöst in 1 ml Acetonitril
Gradienten (alle Angaben in Volumen-%):

| Gradient 1: | | | Gradient 2: | | |
|---|---|---|---|---|---|
| 0 min | 100 % A | 0 % B | 0 min | 100 % A | 0 % B |
| 70 min | 70 % A | 30 % B | 40 min | 70 % A | 30 % B |
| 100 min | 40 % A | 60 % A | 100 min | 40 % A | 60 % B |
| 160 min | 0 % A | 100 % A | 160 min | 0 % A | 100 % B |
| 200 min | 0 % A | 100 % A | 200 min | 0 % A | 100 % B |
| 220 min | 100 % A | 0 % A | 220 min | 100 % A | 0 % B |

Die Proben aus der Aufarbeitung 2.2 oder 2.3 wurden zunächst mit dem Gradienten 1 chromatographiert. Dazu werden 100 mg der Testsubstanz in 1 ml Acetonitril auf die Säule aufgetragen und chromatographiert. Der fast reine Wirkstoff (Omphalotin) kommt zwischen 100 bis 160 min bei 30 % A. Diese Fraktion (= weiter aufgereinigtes Produkt) wurde anschließend im Gradienten 2 rechromatographiert. Der reine Wirkstoff befand sich in der Fraktion, die zwischen 100 bis 160 min erscheint bei 30 % B. Die Fraktion mit reinem Wirkstoff wird durch Destillation im Rotationsverdampfer unter Vakuum vom Lösungsmittel befreit. Ausgehend von 400 g Mycel erhielt man 11,5 mg des reinen Wirkstoffes bei mehrfachen Durchläufen der oben genannten Arbeitsschritte.

### 2.4 Analytische Hochdruckflüssigkeitschromatographie (HPLC)

Gerät: HPLC 1090 mit Diodenarraydetektor der Fa. Hewlett Packard (Waldbronn, Deutschland)
Säule: Lichrocart, Lichrospher RP 18 WP 300® (5 µm Partikeldurchmesser)
Dimension der Säule: 250*4 mm
Eluent: A = Wasser, B = Acetonitril
Fluß: 1 ml/min
Temperatur: 40°C
Detektion: UV, λ = 210 nm
Injektionsmenge: 10 µl einer 0,1%igen Lösung (Gewichtsprozente) des Probengutes in Acetonitril
Gradient:

| Gradient | | |
|---|---|---|
| 0 min | 0 % B | 100 % A |
| 15 min | 60 % B | 30 % A |
| 30 min | 100 % B | 0 % A |
| 35 min | 100 % B | 0 % A |
| 40 min | 0 % B | 100 % A |

Retentionszeit für Omphalotin unter diesen Bedingungen 20,8 min.

Die zu analysierenden Fraktionen werden eingedampft am Rotationsverdampfer, dann in Acetonitril aufgenommen und als 0,1 % Lösung auf die Säule injiziert. Die Retentionszeit für Omphalotin beträgt 20,8 min. Der Nachweis erfolgt über das UV-Spektrum, das vom Gerät automatisch für das Eluat bestimmt wird.

### Beispiel A

### Wirkung von Omphalotin auf Heterodera schachtii

### Testnematode: Heterodera schachtii

Testmethode: Die Prüfsubstanz wird mit Methanol gelöst und in die Vertiefungen von Gewebekulturplatten (24-Lochtablett) pipetiert. Danach läßt man das Methanol vollständig abdampfen. Die Vertiefungen werden mit je 0,8 ml physiologischer Kochsalzlösung gefüllt. Anschließend wird in jede Vertiefung 0,2 ml einer Nemtodensuspension (500 Nematoden / ml) pipetiert.

Nach 24 Stunden Inkubation bei Raumtemperatur und leichtem Schütteln der Platten wird der Anteil der lebenden bzw. toter Nematoden ausgezählt.

Der Wirkungsgrad der Testsubstanz beträgt 100 %, wenn alle Testnematoden getötet worden sind bzw. er beträgt 0 %, wenn noch genauso viele Nematoden leben wie in der Kontrolle.

| Folgende Wirkungsgrade wurden ermittelt: | |
|---|---|
| Konzentration von Omphalotin (ppm = mg/l) | Wirkungsgrad (in % Abbott) |
| 10 ppm | 60 |
| 5 ppm | 40 |

### Beispiel B

### Wirkung von Omphalotin auf Meloidogyne incognita

### Testnematode: Meloidogyne incognita

Testmethode: In eine 24-Lochmikrotiterplatte wird die in Methanol gelöste Testsubstanz einpipettiert und das Lösungsmittel abgedampft. Anschließend werden pro Loch der Mikrotiterplatte 400 µl Nematodensuspension zugegeben. Die Zahl der Nematoden beträgt bei Verwendung von Meloidogyne incognita 100 L2/400 µl. Um die Löslichkeit der Proben im wäßrigen Milieu zu fördern, wird die Platte 15 min bei 100 upm (IKA MTS 4 Plattenschüttler) geschüttelt und anschließend die erste mikroskopische Kontrolle (Leitz Inversmikroskop) durchgeführt. Die Auswertung erfolgt nach 16 h.

| Nematode | Nematizide Aktivität von Omphalotin (µg/ml) | |
|---|---|---|
| | LD 90 | LD 50 |
| Meloidogyne incognita | 1 - 1,5 | 0,75 |

Diese Testmethode kann auch besonders günstig bei der Omphalotin-Herstellung für das Testen von Omphalotin enthaltenden Fraktionen auf ihren Wirkstoffgehalt eingesetzt werden.

### Beispiel C

### Wirkung von Omphalotin auf Meloidogyne incognita im Gewächshaus (Testkultur: Salat)

### Testnematode: Meloidogyne incognita

Testmethode: Die Prüfsubstanz wird als Lösung (10 ml pro 250 ml Boden) homogen in ein Acker-Lauberde-Gemisch eingerührt zusammen mit ca. 700 Nematoden (Gemisch aus Eiern und Larven). Das behandelte und inokulierte Erdsubstrat wird in 7 x 7 x 6 cm große Töpfe gefüllt, auf denen Salat (Sorte Attraktion) ausgesät wird. Die Samen werden angedrückt und leicht mit Quarzsand abgedeckt. Die Töpfe werden im Gewächshaus bei 25°C inkubiert und gleichmäßig feucht gehalten. Nach 24 Tagen erfolgt die Auswertung durch Auswaschen der Wurzeln und Bestimmung der gebildeten Gallen pro Pflanze.

Der Wirkungsgrad der Testsubstanz beträgt 100 %, wenn keine Gallen gebildet worden sind bzw. er beträgt 0 %, wenn noch genauso viele Gallen gebildet wurden wie in der Kontrolle.

| Testsubstanz (Konzentration bezogen auf das Erdvolumen in ppm = mg/l) | Wirkungsgrad (in % Abbott) |
|---|---|
| Omphalotin 0,5 ppm | 48 |
| Omphalotin 2,5 ppm | 96 |

### Beispiel D

### Wirkung von Omphalotin auf Meioidogyne incognita im Gewächshaus (Testkultur: Gurken)

### Testnematode: Meloidogyne incognita

Testmethode: Anzucht von je zwei Gurkenpflanzen (Sorte Bella) in 7 x 7 x 6 cm großen Töpfen in einem Acker-Lauberde-Gemisch. Die Prüfsubstanz wird in Methanol angelöst, dann in Wasser verdünnt und an die 14 Tage alten Gurkenpflanzen gegossen. Pro Topf werden 10 ml Lösung angegossen. 3 Stunden nach der Behandlung werden auf jeden Topf ca. 500 Nematoden als Suspension aufpipettiert. Die Töpfe werden im Gewächshaus bei 28°C Tagestemperatur und 20°C Nachttemperatur inkubiert. Nach 20 Tagen erfolgt die Auswertung durch Auswaschen der Wurzeln und Bestimmung der gebildeten Gallen pro Pflanze.

Der Wirkungsgrad der Testsubstanz beträgt 100 %, wenn keine Gallen gebildet worden sind bzw. er beträgt 0 %, wenn noch genauso viele Gallen gebildet wurden wie in der Kontrolle.

| Testsubstanz (Konzentration bezogen auf das Erdvolumen in ppm = mg/l) | Wirkungsgrad (in % Abbott) |
|---|---|
| Kontrolle Methanol | 0 |
| Omphalotin 5 ppm | 61 |

### Beispiel E

### Wirkung der erfindungsgemäßen Substanz auf Nematoden

### Testnematode: Radophulus similis

### Testmethode

Die Substanz wird in Methanol gelöst. Anschließend werden Verdünnungen hergestellt und in die Vertiefungen einer Multiwell-Gewebekulturplatte (24-Loch) pipettiert. Danach läßt man das Methanol unter dem Abzug verdampfen und füllt 0,8 ml physiologische Kochsalzlösung in die Vertiefungen. Je 100 Nematoden in 0,2 ml Wasser werden dazupipettiert.

Nach 24-stündiger Inkubation bei ca. 22°C erfolgt die Auswertung unter dem Mikroskop, indem der Anteil der toten Nematoden in den Behandlungsvarianten im Vergleich zur unbehandelten Kontrolle ermittelt wird.

Der Wirkungsgrad der Testsubstanz entspricht 100 %, wenn alle Nematoden getötet worden sind. Leben genauso viele Nematoden wie in der Kontrollvariante, so beträgt der Wirkungsgrad 0 %.

| Folgende Wirkungsgrade wurden ermittelt: | |
|---|---|
| Konzentration der erfindungsgemäßen Substanz (ppm = mg/l) | Wirkungsgrad in % (nach Abbott) |
| 1 ppm | 23 |
| 10 ppm | 46 |
| 30 ppm | 54 |

### Beispiel F

### Wirkung der erfindungsgemäßen Substanz auf Nematoden

### Testnematode: Pratylenchus penetrans

### Testmethode:

Die Substanz wird in Methanol gelöst. Anschließend werden Verdünnungen hergestellt und in die Vertiefungen einer Multiwell-Gewebekulturplatte (24-Loch) pipettiert. Danach läßt man das Methanol unter dem Abzug verdampfen und füllt 0,8 ml physiologische Kochsalzlösung in die Vertiefungen. Je 100 Nematoden in 0,2 ml Wasser werden dazupipettiert.

Nach 24-stündiger Inkubation bei ca. 22°C erfolgt die Auswertung unter dem Mikroskop, indem der Anteil der toten Nematoden in den Behandlungsvarianten im Vergleich zur unbehandelten Kontrolle ermittelt wird.

Der Wirkungsgrad entspricht 100 %, wenn alle Nematoden getötet worden sind. Leben genauso viele Nematoden wie in der Kontrollvariante, so beträgt der Wirkungsgrad 0 %.

| Folgende Wirkungsgrade wurden ermittelt: | |
|---|---|
| Konzentration der erfindungsgemäßen Substanz (ppm = mg/l) | Wirkungsgrad in % (nach Abbott) |
| 10 ppm | 67 |
| 30 ppm | 97 |

### Beispiel G

### Wirkung von Omphalotin auf Insekten

### Testinsekt: Plutella xylosella

### Testmethode:

Die Prübsubstanz wird mit Methanol gelöst und auf die Prüfkonzentration mit Emulgatorwasser (0,4 ml Emulgator W/l H₂O) verdünnt. Mit Hilfe eines Korkbohrers werden aus Kohlblättern Scheiben mit 4,4 cm Durchmesser ausgestanzt. Die Blattscheiben werden mittels einer Pinzette in die Lösungen der Prüfsubstanzen getaucht und anschließend in mit Filterpapier ausgelegte Petrischalen (9 cm Durchmesser) gelegt (1 Blattscheibe / Petrischale). Je Blattscheibe wird mit 6 L3-Larven infiziert. Nach zwei, sechs und 16 Tagen wird bonitiert. Am dritten und gegebenenfalls am sechsten Tag nach der Behandlung wird mit unbehandelten Blattscheiben nachgefüttert.

Der Wirkungsgrad der Testsubstanz beträgt 100 %, wenn alle Testinsekten getötet worden sind bzw. er beträgt 0 %, wenn noch genauso viele Insekten leben wie in der Kontrolle.

| Folgende Wirkungsgrade wurden ermittelt: | | |
|---|---|---|
| Konzentration von Omphalotin (ppm = mg/l) | Wirkungsgrad (in % Abbott) | Wirkungsgrad (in % Abbott) |
| | nach 2 Tagen | nach 6 Tagen |
| 100 ppm | 33 | 100 |

### Beispiel H

Wirkung von Omphalotin auf verschiedene Pilze und Bakterien

Testmethode: Petrischalen werden mit 10 ml Potato-Dextrose-Agar ausgegossen. Auf den Agar wird die Testsubstanz als Lösung ausplatiert und so lange mit dem Spatel verteilt bis die Lösung vom Nährmedium aufgesaugt worden ist. Auf die Agarplatten wird das Inokulum der zu testenden Mikroorganismen als Sporen bzw. Mycelfragmente aufgestempelt, indem ein gegebenenfalls mit Filz bezogener Stempel erst auf eine gut bewachsene Platte gedrückt wird und anschließend auf die Testplatte. Nach 5 bis 6 Tagen wird das radiale Wachstum der Mikroorganismen Kolonien gemessen und mit dem der unbehandelten Kontrollen verglichen.

Der Wirkungsgrad der Testsubstanz beträgt 100 %, wenn kein Wachstum gemessen wurde bzw. er beträgt 0 %, wenn noch genauso viel Wachstum gemessen wurde wie in der Kontrolle.

| Mikroorganismus | Wirkungsgrad von Omphalotin (10 ppm (= mg/l) bezogen auf das Nährmediumsvolumen) |
|---|---|
| Fusarium culmorum | 51 |
| Pythium ultimum | 25 |
| Fusarium graminearum | 23 |
| Alternaria mali | 70 |
| Rhizoctonia solani | 90 |
| Septoria nodorum | 60 |
| Phytophthora cactorum | 50 |
| Pseudocercosporella herpotrichoides | 55 |
| Pyricularia oryzae | 70 |
| Xanthomonas versicatoria | 95 |

Diese Testmethode kann auch in besonders vorteilhafter Weise bei der Herstellung von Omphalotin eingesetzt werden, um die Omphalotin enthaltenden Fraktionen zu bestimmen.

## Patentansprüche

1. Organisch-chemische Verbindung (Omphalotin) der Formel (I) 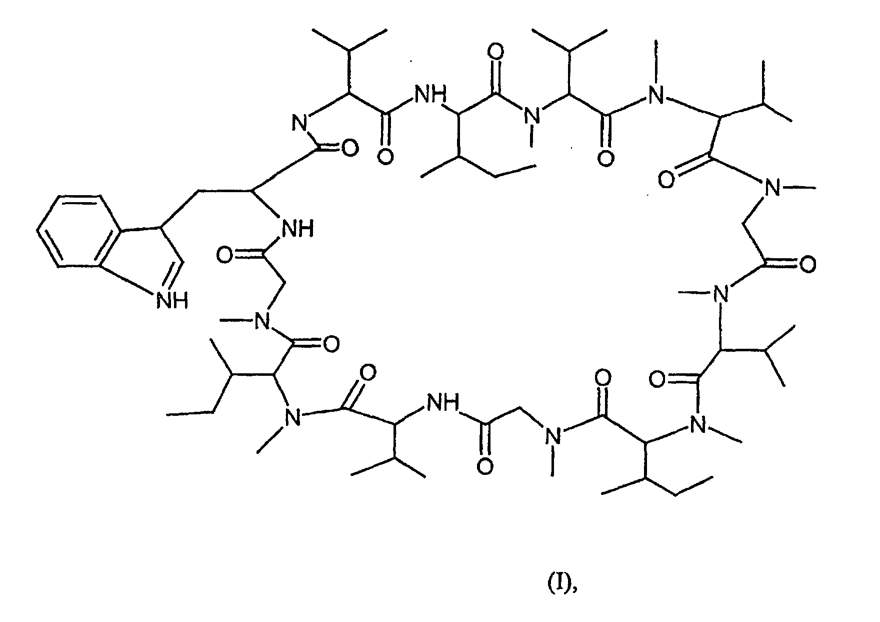 wobei ― (endständig) für -CH₃, 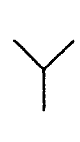 für 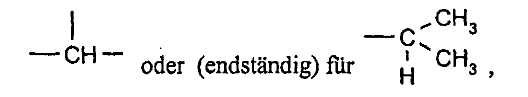 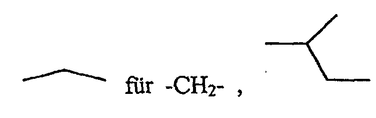 für 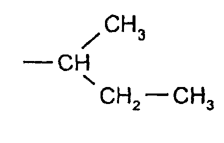 und >=O für 〉C=O stehen.

2. Organisch-chemische Verbindung (Omphalotin), die bei der Fermentation von Omphalotus olearius-Stämmen entsteht und welche die folgenden physikalisch chemischen Daten und Parameter aufweist:
| | | |
|---|---|---|
| a) | Aussehen | weißes Pulver |
| | | |
| b) | Löslichkeit | leicht löslich in Methanol, Aceton und 2-Propanol, weniger löslich in Essigsäureethylester und schwer löslich in Cyclohexan und t.-Butyl-methylether |
| | | |
| c) | Molgewicht | 1317 |
| | | |
| d) | Summenformel (Elementaranalyse) | C₆₉H₁₁₅N₁₃O₁₂ |
| | | |
| e) | Spektren | Die Daten der ¹H-NMR-Spektren sind aus den Tabellen 1 und 2 der Beschreibung ersichtlich |

3. Verfahren zur Herstellung der organisch-chemischen Verbindung (Omphalotin) gemäß Anspruch 1 oder 2, **dadurch gekennzeichnet, dass** man hierfür den Omphalotus olearius-Stamm Nr. 83 039 (gemäß DSM 9737), 90 173 (gemäß DSM 9738), 91 050 (gemäß DSM 9739), 92 095 (gemäß DSM 9740), 93 162 (gemäß DSM 9741) oder 90 170 (gemäß DSM 9742) oder entsprechende Mutanten und Varianten in einem Nährmedium, welches assimilierbare Kohlenstoff- und Stickstoffquellen sowie Mineralsalze enthält unter aeroben Bedingungen kultiviert und die gewünschte Verbindung nach allgemein üblichen Methoden isoliert.

4. Organisch-chemische Verbindung gemäß Anspruch 1 oder 2, erhältlich nach dem Verfahren gemäß Anspruch 3.

5. Schädlingsbekämpfungsmittel, **gekennzeichnet durch** einen Gehalt an der organisch-chemischen Verbindung gemäß Anspruch 1, 2 oder 4.

6. Verwendung der organisch-chemischen Verbindung gemäß Anspruch 1, 2 oder 4 zur Bekämpfung von Schädlingen, insbesondere von Insekten, Nematoden und Mikroben.

7. Verfahren zur Bekämpfung von Schädlingen, **dadurch gekennzeichnet, dass** man die organisch-chemische Verbindung gemäß Anspruch 1, 2 oder 4 auf Schädlinge, vorzugsweise Insekten, Nematoden und Mikroben oder ihren Lebensraum einwirken lässt.

8. Verfahren zur Herstellung von Schädlingsbekämpfungsmitteln, **dadurch gekennzeichnet, dass** man die organisch-chemische Verbindung gemäß Anspruch 1, 2 oder 4 mit Streckmitteln und/oder oberflächenaktiven Mitteln vermischt.

9. Omphalotus olearius-Stamm Nr. 83 039 (gemäß DSM 9737), 90 173 (gemäß DSM 9738), 91 050 (gemäß DSM 9739), 92 095 (gemäß DSM 9740), 93 162 (gemäß DSM 9741) oder 90 170 (gemäß DSM 9742) sowie deren Mutanten und Varianten.

10. Organisch-chemische Verbindung gemäß Anspruch 1, 2 oder 4 zur Bekämpfung von Erkrankungen, welche durch Parasiten, vorzugsweise Nematoden, oder Mikroben, vorzugsweise Pilze und Bakterien, hervorgerufen werden.

## Claims

1. Organochemical compound (omphalotin) of the formula (I) 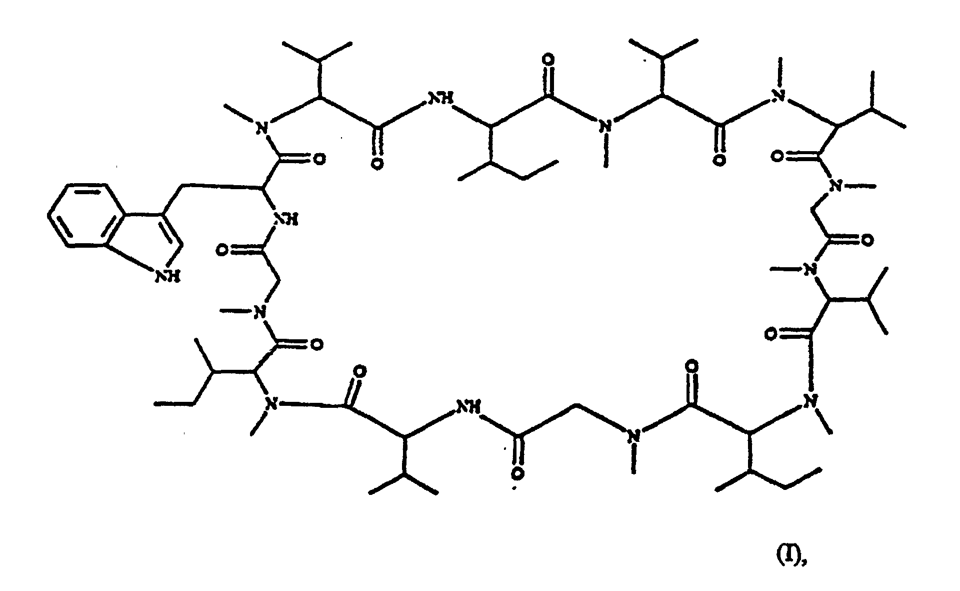 where ― (terminal) represents -CH₃, 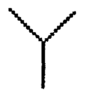 represents 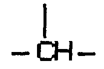 or (terminal) represents 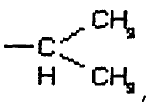 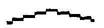 represents -CH₂- , 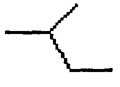 represents 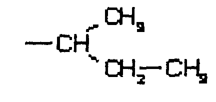 and >=O represents 〉C=O.

2. Organochemical compound (omphalotin) which is formed during the fermentation of Omphalotus olearius strains and which has the following physico-chemical data and parameters:
| | | |
|---|---|---|
| a) | Appearance | white powder |
| | | |
| b) | Solubility | readily soluble in methanol, acetone and 2-propanol, less readily soluble in ethyl acetate and hardly soluble in cyclohexane and t-butyl methyl ether |
| | | |
| c) | Molecular weight | 1317 |
| | | |
| d) | Empirical formula (elemental analysis) | C₆₉H₁₁₅N₁₃O₁₂ |
| | | |
| e) | Spectra | The data of the ¹H NMR spectra are shown in Tables 1 and 2 of the description. |

3. Process for preparing the organochemical compound (omphalotin) according to Claims 1 or 2, **characterized in that** the Omphalotus olearius strain No. 83 039 (according to DSM 9737), 90 173 (according to DSM 9738), 91 050 (according to DSM 9739), 92 095 (according to DSM 9740), 93 162 (according to DSM 9741) or 90 170 (according to DSM 9742) or corresponding mutants and variants are cultivated under aerobic conditions in a culture medium which contains assimilable carbon and nitrogen sources and mineral salts, and the desired compound is isolated by customary methods.

4. Organochemical compound according to Claims 1 or 2, obtainable by the process according to Claim 3.

5. Pesticide, **characterized in that** it contains the organochemical compound according to Claim 1, 2 or 4.

6. The use of the organochemical compound according to Claim 1, 2 or 4 for controlling pests, in particular insects, nematodes and microbes.

7. Method for controlling pests, **characterized in that** the organochemical compound according to Claim 1, 2 or 4 is allowed to act on pests, preferably on insects, nematodes and microbes, or their habitat.

8. Process for preparing pesticides, **characterized in that** the organochemical compound according to Claim 1, 2 or 4 is mixed with extenders and/or surfactants.

9. Omphalotus olearius strain No. 83 039 (according to DSM 9737), 90 173 (according to DSM 9738), 91 050 (according to DSM 9739), 92 095 (according to DSM 9740), 93 162 (according to DSM 9741) or 90 170 (according to DSM 9742) and mutants and variants thereof are employed.

10. Organochemical compound according to Claim 1, 2 or 4 for controlling diseases which are caused by parasites, preferably nematodes, or microbes, preferably fungi and bacteria.

## Revendications

1. Composé organochimique (omphalotine) de formule (I) 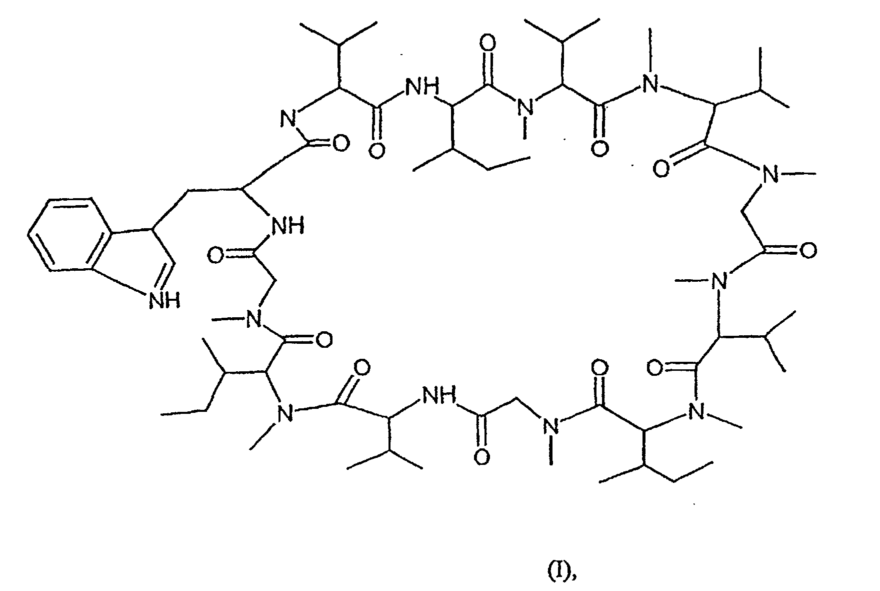 dans laquelle ― (en position terminale) représente -CH₃, 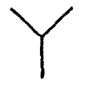 représente 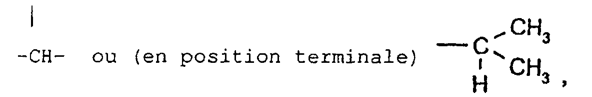 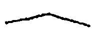 représente -CH₂-, 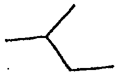 représente 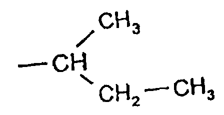 et >=O représente 〉C=O.

2. Composé organochimique (omphalotine) qui est produit au cours de la fermentation de souches d'Omphalotus olearius et qui présente les caractéristiques et paramètres physico-chimiques suivants :
| | | |
|---|---|---|
| a) | Aspect | poudre blanche |
| | | |
| b) | Solubilité | très soluble dans le méthanol, l'acétone et le 2-propanol, moins soluble dans l'ester éthylique d'acide acétique et très peu soluble dans le cyclohexane et l'éther de tertio-butyle et de méthyle |
| | | |
| c) | poids moléculaire | 1317 |
| | | |
| d) | Formule brute (analyse élémentaire) | C₆₉H₁₁₅N₁₃O₁₂ |
| | | |
| e) | Spectres | Les caractéristiques des spectres de ¹H-RMN ressortent des tableaux 1 et 2 de la description. |

3. Procédé de production du composé organochimique (omphalotine) suivant la revendication 1 ou 2, **caractérisé en ce qu'**on cultive à cet effet dans des conditions aérobies la souche d'Omphalotus olearius N° 83 039 (conformément à DSM 9737), N° 90 173 (conformément à DSM 9738), N° 91 050 (conformément à DSM 9739), N° 92 095 (conformément à DSM 9740), N° 93 162 (conformément à DSM 9741) ou N° 90 170 (conformément à DSM 9742) ou des mutants ou variants correspondants dans un milieu nutritif qui contient des sources assimilables de carbone et d'azote ainsi que des sels minéraux et on isole le composé voulu selon des méthodes généralement classiques.

4. Composé organochimique suivant la revendication 1 ou 2, pouvant être obtenu par le procédé selon la revendication 3.

5. Composition pesticide, **caractérisée par** une teneur en le composé organochimique suivant la revendication 1, 2 ou 4.

6. Utilisation du composé organochimique suivant la revendication 1, 2 ou 4 pour combattre des parasites, en particulier des insectes, des nématodes et des micro-organismes.

7. Procédé pour combattre des parasites, **caractérisé en ce qu'**on fait agir le composé organochimique suivant les revendications 1, 2 ou 4 sur des parasites, principalement des insectes, des nématodes et des micro-organismes, ou sur leur milieu.

8. Procédé de préparation de compositions pesticides, **caractérisé en ce qu'**on mélange le composé organochimique suivant la revendication 1, 2 ou 4 avec des diluants et/ou des agents tensio-actifs.

9. Souche d'Omphalotus olearius N° 83 039 (conformément à DSM 9737), N° 90 173 (conformément à DSM 9738), N° 91 050 (conformément à DSM 9739), N° 92 095 (conformément à DSM 9740), N° 93 162 (conformément à DSM 9741) ou N° 90 170 (conformément à DSM 9742) ainsi que les mutants et variants de cette souche.

10. Composé organochimique suivant la revendication 1, 2 ou 4, destiné à combattre des maladies qui sont causées par des parasites, principalement des nématodes, ou des micro-organismes, principalement des champignons et des bactéries.
